# EUROPEAN PATENT SPECIFICATION

(11) **EP 1 323 834 B1**
(45) Date of publication and mention of the grant of the patent: **05.07.2006**
(21) Application number: 02028697.7
(22) Date of filing: 23.12.2002
(51) Int. Cl.: C12Q 1/68, G01N 33/543

(54) **Target detection method, reagent and method for detecting SNP of DNA nucleotide sequences**
Detektionsmethode für Zielmoleküle, Reagenz sowie Verfahren zur Detektion von SNPs in DNA Nukleotidsequenzen
Procédé pour détecter des molécules cibles, réagent et procédé pour la détection des SNP dans des séquences d'acides nucléiques

(30) Priority: 25.12.2001 JP 2001392018
(43) Date of publication of application: 02.07.2003
(73) Proprietor: HITACHI SOFTWARE ENGINEERING CO., LTD., Yokohama-shi, Kanagawa 230-0045 (JP)
(72) Inventor: Sasaki, Kohsuke, c/o Yamaguchi University School, Ube-shi, Yamaguchi 755-8505 (JP); Furuya, Tomoko, c/o Yamaguchi University School, Ube-shi, Yamaguchi 755-8505 (JP)
(74) Representative: Liesegang, Roland

(56) References cited:
- WO-A-93/02360
- US-A- 5 981 180
- US-A- 6 159 748
- WEDEMEYER N. ET AL.: "Flow cytometry: an 'old' tool for novel applications in medical genetics" CLIN GENET, vol. 60, July 2001 (2001-07), pages 1-8, XP002259894 Munksgaard
- ARMSTRONG B ET AL: "SUSPENSON ARRAYS FOR HIGH THROUGHPUT, MULTIPLEXED SINGLE NUCLEOTIDE POLYMORPHISM GENOTYPING" CYTOMETRY, ALAN R. LISS, INC, XX, vol. 40, no. 2, 1 June 2000 (2000-06-01), pages 102-108, XP001106772 ISSN: 0196-4763
- LANDEGREN U ET AL: "Reading bits of genetic information: methods for single-nucleotide polymorphism analysis" GENOME RESEARCH, COLD SPRING HARBOR LABORATORY PRESS, US, vol. 8, no. 8, August 1998 (1998-08), pages 769-776, XP002223654 ISSN: 1088-9051
- LEWIN, B.: "Genes IV" 1990 , OUP , OXFORD XP002259895 * page 811, column 2 *
- LACKNER K J ET AL: "MULTIPLEX DNA-UND RNA-ANALYSE AN FLUORESZENTEN MICROBEADS ALS ALTERNATIVE ZUM DNA-ARRAY" MEDIZINISCHE GENETIK, BERUFSVERBAND MEDIZINISCHE GENETIK, MUNCHEN,, DE, vol. 11, 1999, pages 16-17, XP000930079 ISSN: 0936-5931
- FULTON R J ET AL: "Advanced multiplexd analysis with the FlowMetrix(TM) system" CLINICAL CHEMISTRY, AMERICAN ASSOCIATION FOR CLINICAL CHEMISTRY. WINSTON, US, vol. 43, no. 9, September 1997 (1997-09), pages 1749-1756, XP002142645 ISSN: 0009-9147

### SEQUENCE LISTING

<110> Hitachi Software Engineering Co., Ltd.
<120> TARGET DETECTION METHOD, REAGENT AND METHOD FOR DETECTING SNP OF DNA NUCLEOTIDE SEQUENCES
<130> PH-1688
<140>JP2001-392018
   <141>2001-12-25
<160> 10
<170> Patent In Ver. 2.1
<210> 1
   <211> 17
   <212> DNA
   <213> Artificial Sequence
<220>
   <223> Description of Artificial Sequence: Synthetic DNA
<400> 1
   tccttcgctc atttcag 17
<210> 2
   <211> 17
<212> DNA
   <213> Artificial Sequence
<220>
   <223> Description of Artificial Sequence: Synthetic DNA
<400> 2
   tccttcgcgc atttcag 17
<210> 3
   <211> 17
   <212> DNA
   <213> Artificial Sequence
<220>
   <223> Description of Artificial Sequence: Synthetic DNA
<400> 3
   tgcattaccg gaagtgg 17
<210> 4
   <211> 17
   <212> DNA
   <213> Artificial Sequence
<220>
   <223> Description of Artificial Sequence: Synthetic DNA
<400> 4
   tgcattactg gaagtgg 17
<210> 5
   <211> 17
   <212> DNA
   <213> Artificial Sequence
<220>
   <223> Description of Artificial Sequence: Synthetic DNA
<400> 5
   tcgctggcgt gaaggac 17
<210> 6
   <211> 17
   <212> DNA
   <213> Artificial Sequence
<220>
   <223> Description of Artificial Sequence: Synthetic DNA
<400> 6
   tcgctggcat gaaggac 17
<210> 7
   <211> 24
   <212> DNA
   <213> Artificial Sequence
<220>
   <223> Description of Artificial Sequence: Synthetic DNA
<400> 7
   taaactcttt ccaggatggc ctgc 24
<210> 8
   <211> 24
   <212> DNA
   <213> Artificial Sequence
<220>
   <223> Description of Artificial Sequence: Synthetic DNA
<400> 8
   accatgcccc caagtgctaa gaag 24
<210> 9
   <211> 24
   <212> DNA
   <213> Artificial Sequence
<220>
   <223> Description of Artificial Sequence: Synthetic DNA
<400> 9
   atcacccagc ggatggtgga tttc 24
<210> 10
   <211> 24
   <212> DNA
   <213> Artificial Sequence
<220>
   <223> Description of Artificial Sequence: Synthetic DNA
<400> 10
   tttttccagg tctgacaacg ggtc 24

## Description

### BACKGROUND OF THE INVENTION

### 1. Technical Field

The present invention relates to a method of detecting a target sequence by using beads such as polystyrene beads, polypropylene beads, magnetic beads, and glass beads. The invention also relates to a reagent and method for detecting single nucleotide polymorphisms (SNPs) of DNA nucleotide sequences.

### 2. Background Art

A biochip (DNA microarray) is used for detecting many kinds of biopolymers, such as DNA nucleotide sequences, contained in a sample at once. The biochip takes advantage of the fact that multiple kinds of labeled targets (such as DNAs) in a sample selectively bind to multiple kinds of probes (such as DNAs) by hybridization, the probes being immobilized on a flat supporter such as a slide glass. Specifically, a target sequence in the sample is detected by means of a marker that is introduced as the target hybridizes to a spot where a probe is immobilized. By immobilizing a great number of kinds of probes on a single supporter, many kinds of targets can be detected at the same time.

Another hybridization method that has been developed uses spherical beads instead of the flat supporter. Each bead has a diameter of the order of from 100nm to 100µm. Probes are immobilized on the surface of the beads, so that the probes are hybridized with targets on the surface of the beads. This method, which uses the surfaces of the spheres as the site of reaction, has the advantage that the reaction efficiency and cleaning efficiency are high.

Although the method of hybridizing a probe with a target on the bead surface has a high reaction efficiency, the target that has hybridized to the probe immobilized on the beads surface must be identified by identifying the bead itself. For this reason, when there is only one kind of beads, only one kind of target can be identified. Another method is known which employs multiple kinds of beads that are made identifiable by coloring them with fluorescent pigments. But this method requires a separate laser light source for exciting the fluorescent pigments that color the beads, in addition to the laser light source for exciting the fluorescent pigments labeling the targets. As a result, the system becomes complex and expensive.

### SUMMARY OF THE INVENTION

In view of those problems of the prior art mentioned above, it is an object of the invention to provide a target detection method that employs multiple kinds of beads on which different probes are immobilized, and that enables the beads themselves to be identified in a simple manner. The invention also provides a reagent and method for detecting SNPs of a DNA nucleotide sequence by using a single kind of beads with the same grain size.

The above-mentioned object is achieved by the invention employing beads of different grain sizes that are flown in a flow cytometer to measure forward scatter. Based on the intensity of the forward scatter, the grain sizes of the beads are identified. Different probes are immobilized on the surface of the beads of different grain sizes. After hybridizing the probes with a fluorescence-labeled target, the fluorescence intensity of the beads are measured by the flow cytometer to detect the target.

The above object is also achieved as follows. Beads on which a probe is immobilized that hybridizes with a DNA nucleotide sequence having no SNP is mixed with beads on which another probe is immobilized that hybridizes with a DNA nucleotide sequence having SNP to make a reagent. The reagent is added to a sample solution containing a fluorescence-labeled DNA nucleotide sequence under investigation and a hybridization reaction is effected. The beads after the reaction are flown in a flow cytometer to obtain a fluorescence intensity histogram pattern, based on which SNP of the DNA nucleotide sequences under investigation is determined.

In one aspect, the invention provides a target detection method comprising the steps of:
effecting a hybridization reaction between a target and a probe by mixing, in a sample solution containing a fluorescence-labeled target, beads of a first grain size having a first probe immobilized on the surface thereof and beads of a second grain size having a second probe which is different from the first probe immobilized on the surface thereof;
detecting, after the hybridization reaction, forward scatter and fluorescence simultaneously by flowing the beads in a flow cytometer; and
identifying the grain size of the beads based on the intensity of the forward scatter.

Beads of the same grain size may have the same probe immobilized thereon, and beads of different grain sizes may have different probes immobilized thereon.

In another aspect, the invention provides a reagent for detecting SNP in a DNA nucleotide sequence, comprising a mixture of first and second beads, wherein the first beads have a probe immobilized on the surface thereof which hybridizes with a DNA nucleotide sequence having no SNP, wherein the second beads differ from the first beads only in that the sequence of the probe immobilized on the surface of the second beads hybridizes with a DNA nucleotide sequence having SNP.

The first beads and the second beads may be mixed either at the same proportions or at different proportions.

In yet another aspect, the invention provides a reagent for detecting SNPs in a DNA nucleotide sequence, which comprises a mixture of:
first beads of a first grain size having a probe immobilized on the surface thereof, the probe hybridizing with a first DNA nucleotide sequence having no SNP;
second beads which differ from the first beads only in that the sequence of a probe immobilized on the surface of the second beads hybridizes with the first DNA nucleotide sequence having SNP;
third beads of a second grain size having a probe immobilized on the surface thereof, the probe hybridizing with a second DNA nucleotide sequence having no SNP; and
fourth beads which differ from the third beads only in that the sequence of a probe immobilized on the surface of the fourth beads hybridizes with the second DNA nucleotide sequence having SNP.

The first and second beads are preferably mixed at different proportions, and the third and fourth beads are preferably mixed at different proportions.

The grain size of the beads may be in the range of from 0.1 µm to 1 mm.

In still another aspect, the invention provides a method of detecting SNP in DNA nucleotide sequences, which comprises the steps of:
effecting a hybridization reaction by mixing, in a sample solution containing a fluorescence-labeled DNA nucleotide sequence under investigation, first beads and second beads, the first beads having a probe immobilized on the surface thereof which hybridizes with a DNA nucleotide sequence having no SNP, the second beads differing from the first beads only in that the sequence of a probe immobilized on the surface of the second beads hybridizes with a DNA nucleotide sequence having SNP;
creating a fluorescence intensity histogram by flowing the beads, after the hybridization reaction, in a flow cytometer and measuring the fluorescence intensity; and
determining SNP in the DNA nucleotide sequence under investigation based on a peak pattern in the fluorescence intensity histogram.

The determination of SNP in the DNA nucleotide sequence under investigation may be based on whether the number of the peak is one or two.

The first and second beads are preferably mixed at different proportions. In this case, the SNP in the DNA nucleotide sequence under investigation may be determined based on the ratio of the two peaks with different fluorescence intensities in the fluorescence intensity histogram.

The material of the beads is not particularly limited. For example, polystyrene beads, polypropylene beads, magnetic beads, and glass beads may be used. By setting up filters adapted for different grain sizes of the beads at the discharge portion of the flow cytometer, the beads of different grain sizes can be recovered.

### BRIEF DESCRIPTION OF THE DRAWINGS

Fig. 1 shows the results of measuring the forward scatter from beads with a grain size of 4.5µm.
Fig. 2 shows the results of measuring the forward and side scatter from beads with different grain sizes (4.5µm, 6.0µm and 10.0µm).
Figs. 3A to 3C show histograms of fluorescence intensity of the beads with different diameters after hybridization.
Fig. 4 shows the results of measuring the forward scatter from beads with grain size of 4.5µm and beads with grain size of 6.0µm.
Figs. 5A and 5B show histograms of fluorescence intensity after hybridization of a PCR product with two kinds of beads.
Figs. 6A to 6C show examples of histograms of fluorescence intensity for determining SNPs.
Figs. 7A to 7F show other examples of histograms of fluorescence intensity for determining SNPs.
Fig. 8 schematically shows a system for recovering beads and biopolymers interacting on the surface of the beads.

### DETAILED DESCRIPTION OF PREFERRED EMBODIMENTS

The invention will be hereafter described by way of embodiments with reference made to the attached drawings.

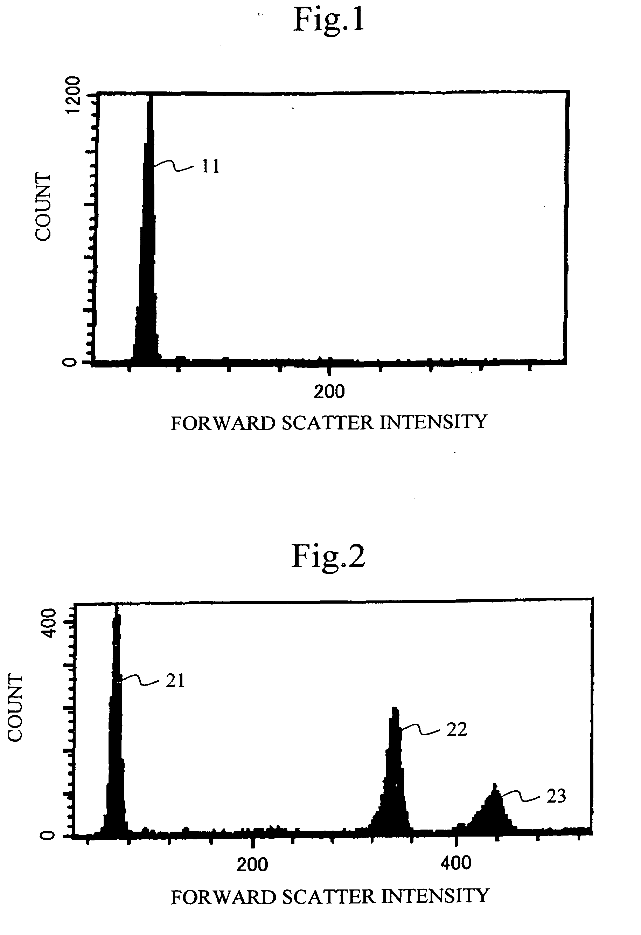

Fig. 1 is a flow cytogram showing the results of measuring the forward scatter as polystyrene beads with a grain size of 4.5µm were flown in a flow cytometer. The horizontal axis indicates the intensity of the forward scatter (in arbitrary units), and the vertical axis indicates the number of beads counted. A steep peak 11 of counts is formed at a specific intensity of forward scatter. This shows that forward scatter of substantially the same intensity is observed when beads with the same grain size intersect the laser light of the flow cytometer. The sum of the counts in the peak 11 corresponds to the total number of the beads detected. Thus, the forward scatter intensity reflects the size (grain size) of the beads or cells, so that the grain size of beads can be identified based on the forward scatter intensity.

Various beads having a carboxyl group on the surface with grain sizes ranging from 0.05 to 90µm are commercially available from Polysciences Inc. or Bangs Laboratories, Inc., for example. A protein, which does not form part of the invention, or a DNA or DNA fragment whose terminal is modified by amination can be passively adsorbed or covalently bonded to the surface of these beads. Beads with a probe such as a DNA or a protein (such protein not forming part of the invention) coupled to the surface thereof are mixed in a sample solution containing a target such as a fluorescence-labeled DNA or protein, thus specifically (such protein not forming part of the invention) hybridizing the target to the probe on the surface of the beads. In this way, mutations in DNA can be detected by utilizing hybridization between DNA and DNA (including RNA, not forming part of the invention, (ribonucleic acid), PNA, not forming part of the invention, (peptide nucleic acid) and LNA (locked nucleic acid), not forming part of the invention, or interactions between various biopolymers, such as between protein and protein, not forming part of the invention, protein and DNA, not forming part of the invention, or sugars, not forming part of the invention, can be analyzed.

The method according to the invention will be described in detail. Three kinds of polystyrene beads with grains sizes of 4,5 µm, 6.0µm and 10.0µm were prepared. DNAs (probes) as shown in Table 1 below were covalently bonded to the surface of the individual beads. Specifically, amino-substituted oligonucleotides (DNAs) were covalently bonded, by means of EDC (ethylenediamine carbodiimide), to the carboxylated beads.

**Table 1**

| Type of bead (grain size) | Name of the probe | Probe sequence |
|---|---|---|
| Polystyrene bead (4.5µm) | UGT normal | TCC/TTC/GCT/CAT/TTC/AG |
| | UGT variant | TCC/TTC/GCG/CAT/TTC/AG |
| Polystyrene bead (6.0µm) | KLK2 normal | TGC/ATT/ACC/GGA/AGT/GG |
| | KLK2 variant | TGC/ATT/ACT/GGA/AGT/GG |
| Polystyrene bead (10.0µm) | Comt normal | TCG/CTGJGCG/TGA/AGG/AC |
| | Comt variant | TCG/CTG/GCA/TGA/AGG/AC |

For each grain size, beads to which a normal probe was covalently bonded and beads to which a variant probe was covalently bonded were prepared, and the two kinds of beads were mixed in equal amounts. The resultant beads with the respective grain sizes were flown in a flow cytometer. Fig. 2 shows the flow cytogram obtained by measuring the forward scatter. The horizontal axis indicates the forward scatter intensity (in arbitrary units), and the vertical axis indicates the counts of beads detected.

As mentioned above, the forward scatter intensity varies depending on the grain size of the beads. Peak 21 is due to the polystyrene beads of 4.5µm grain size. Peak 22 is due to polystyrene beads of 6.0µm grain size. Peak 23 is due to polystyrene beads of 10.0µm grain size. Thus, the grain sizes of the beads can be identified based on the forward scatter intensities measured by the flow cytometer.

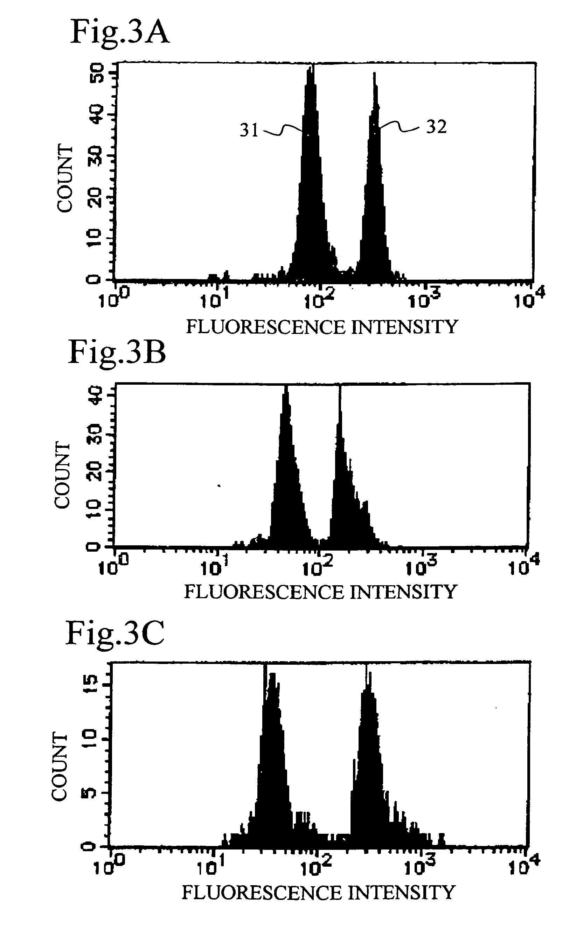

Next, a complementary strand to the normal probe whose terminal was fluorescence-labeled by fluorescein isothiocyanate (FITC) was synthesized. The complementary strand was then hybridized to the beads of the respective grain sizes in the equal-amount mixture of the beads to which the normal probe was covalently bonded and the beads to which the variant probe was covalently bonded. The beads of the respective grain sizes after hybridization were flown to the flow cytometer. Figs. 3A to 3C show fluorescence intensity histograms obtained by measuring the fluorescence intensities. Fig. 3A shows the fluorescence intensity histogram for the polystyrene beads of 4.5µm grain size. Fig. 3B shows the fluorescence intensity histogram for the polystyrene beads of 6.0µm grain size. Fig. 3C shows the fluorescence intensity histogram for the polystyrene beads of 10.0µm grain size. In each histogram, the horizontal axis indicates the fluorescence intensity, and the vertical axis indicates the number of beads counted.

For each grain size, the FITC-labeled complementary strand hybridizes to the beads to which the normal probe was covalently bonded, while, with regard to the variant probe, the hybridization signal was significantly lowered due to the difference in one base. This resulted in the two distinct peaks that are visible in the histograms. For example, peak 32, indicating a strong fluorescence, is due to the beads with the normal probe to which the FITC-labeled complementary strand was hybridized. On the other hand, peak 31 is due to the beads to which the variant probe was covalently bonded. There, because the complementary strand to the fluorescence-labeled normal probe was not hybridized, little fluorescence is observed. In Figs. 3B and 3C too, the right-hand side peak where a strong fluorescence intensity is observed is due to the beads with the normal probe to which the FITC-labeled complementary strand was hybridized, and the left-hand side peak with a weak fluorescence intensity is due to the beads to which the variant probe was covalently bonded.

Next, instead of the synthetic oligo DNA target, a PCR product was hybridized to the beads of the 4.5µm and 6.0µm diameters in which the beads to which the normal probe was covalently bonded and the beads to which the variant probe was covalently bonded were mixed in equal amounts. The PCR product was labeled by FITC. The primers used for PCR were:
UGT forward (TAA/ACT/CTT/TCC/AGG/ATG/GCC/TGC)
UGT reverse (ACC/ATG/CCC/CCA/AGT/GCT/AAG/AAG)
comt forward (ATC/ACC/CAG/CGG/ATG/GTG/GAT/TTC) and
comt reverse (TTT/TTC/CAG/GTC/TGA/CAA/CGG/GTC).
PCR amplification was performed on 100-mer genome DNA including two SNPs detection sites. The PCR reaction condition was such that after 10 min thermal denaturation at 95°C, a cycle of PCR reaction at 94°C for 15 sec, 55°C for 30 sec, and 68°C for 30 sec was repeated 35 times. DNA was fluorescence-labeled by using FITC-labeled dUTP as dNTP during PCR, and the fluorescence-labeled DNA was used for hybridization.

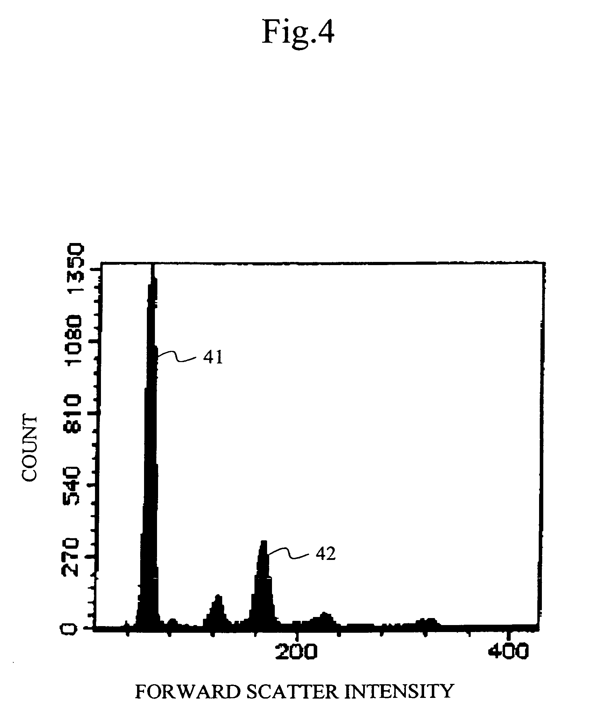

Fig. 4 shows the results of measuring forward scatter from beads of 4.5µm grain size and beads of 6.0µm grain size. Peak 41 with a weaker intensity indicates the forward scatter that is due to the beads of 4.5µm grain size. Peak 42 with a stronger intensity indicates the forward scatter that is due to the beads of 6.0µm grain size.

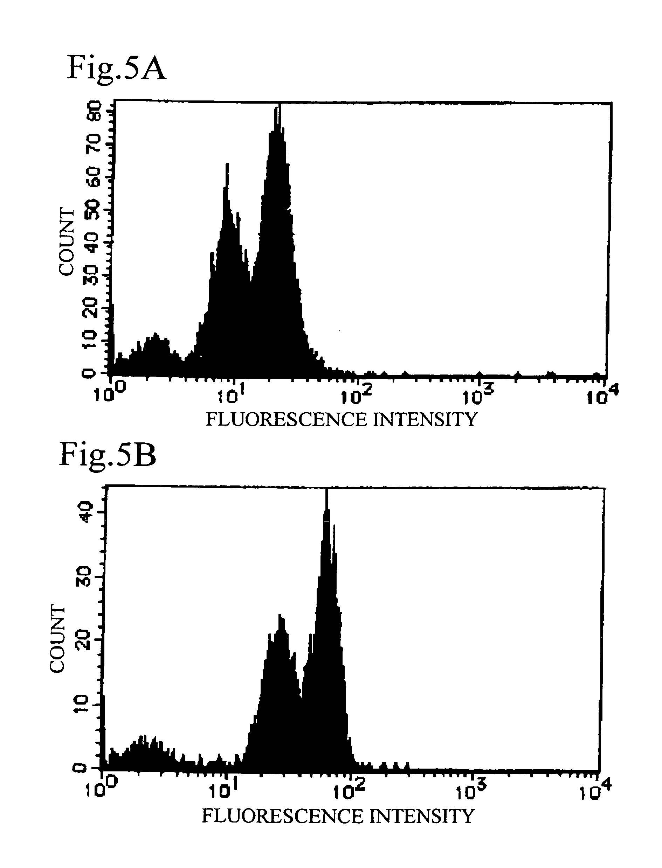

After hybridizing the above-mentioned PCR product (target) to the two kinds of beads of grains sizes 4.5µm and 6.0µm, the beads were flown in the flow cytometer to measure fluorescence intensities, based on which fluorescence intensity histograms were created. Fig. 5A shows a fluorescence intensity histogram for the beads of 4.5µm grain size, while Fig. 5B shows a fluorescence intensity histogram for the beads of 6.0µm grain size.

Both Figs. 5A and 5B show two peaks in the respective fluorescence intensity histograms. This indicates that there is a mixture of those beads that emit fluorescence because of the bonding of the fluorescence-labeled PCR product thereto by hybridization, and those beads that do not emit fluorescence because of the absence of bonding of the PCR product. Namely, the fluorescence intensity histogram of Fig. 5A or 5B is providing the information that the target bonded with either the normal or variant probe and not both. If the target had bonded with both the normal and variant probes, the fluorescence marker would have been introduced into all of the beads, and only a single peak would have appeared in the fluorescence intensity histogram in the flow cytometer at the position corresponding to the above-mentioned peak with a greater intensity. Thus, in accordance with the invention, the information about SNPs can be obtained based on the number of peaks that appear in the fluorescence intensity histograms in the flow cytometer.

The flow cytometer can measure the forward scatter and fluorescence simultaneously for each of the beads that are successively flown. Thus, the flow cytometer stores a pair of the data about forward scatter intensity and the data about fluorescence intensity that are measured as each bead is passed through the detector in the flow cytometer. Thereafter, the data is classified according to the magnitude (corresponding to the grain size of the bead) of forward scatter intensity as shown in Figs. 2 and 4. For each set of data with the same forward scatter intensity, a histogram, such as ones shown in Figs. 3A, 3B, 5A and 5B is created. In this way, the information about how the target is hybridized to the different probes that are bound to the beads of the same grain size, that is the information about whether the target is of homo type or hetero type, can be obtained. By associating beads of one grain size with one gene or one DNA nucleotide sequence, SNPs of multiple genes or DNA nucleotide sequences can be simultaneously detected by using beads of multiple grain sizes.

Hereafter, a method of obtaining more detailed information, such as the kind of the substituting base, in addition to the information about whether the SNP of a DNA nucleotide sequence is of homo or hetero type, will be described. The following example employs one kind of beads of the same grain size to detect SNP of a specific gene (at a single site).

Initially, with regard to a gene of interest, beads A and beads B were prepared. Beads A had a probe covalently bonded to the surface thereof to which a nucleotide sequence having no SNPs would hybridize. Beads B had a probe covalently bonded to the surface thereof to which a nucleotide sequence of SNP would hybridize. Beads A and beads B are mixed at different proportions, such as A:B=1:2. A region of interest in a gene collected from a subject is labeled with a fluorescence marker and PCR-amplified to prepare a sample. This sample is added to the mixture of beads A and beads B and hybridized. Thereafter, the beads having the probe on the surface thereof to which a target in the sample hybridized are measured by a flow cytometer.

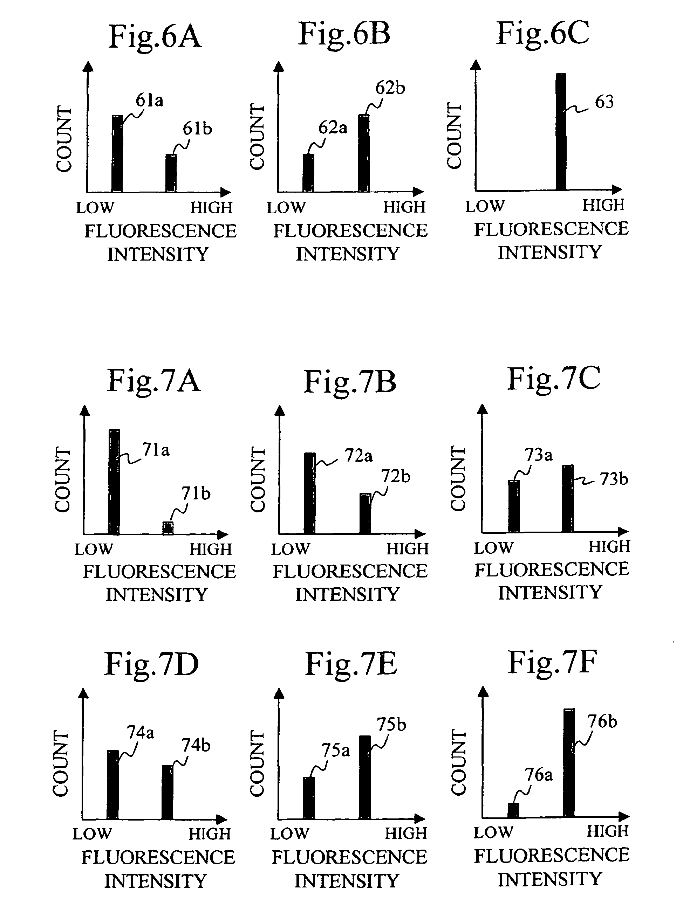

Figs. 6A to 6C show possible three patterns of the resulting fluorescence intensity histograms. Fig. 6A shows the pattern in the case where the fluorescence marker was introduced into beads A that are fewer than beads B, namely when there is no SNPs in the gene of the subject. When the mixture ratio of beads A and beads B is such that A:B=1:2, ideally the ratio of the count for peak 61a and the count for peak 61b would be 2:1. Fig. 6B shows the pattern in the case where the fluorescence marker was introduced into beads B that are more than beads A, namely when there is SNP in the gene of the subject. In this case, ideally the ratio of the count for peak 62a and the count for peak 62b would be 1:2. Fig. 6C shows the pattern (hetero type) in the case where the fluorescence marker was introduced into both beads A and beads B, namely when the gene in the subject has both nucleotide sequences without SNPs and nucleotide sequences with SNPs. In this case, there is only one peak 63 in the fluorescence intensity histogram.

Thus, by performing the above examination and determining to which one of Figs. 6A to 6C the pattern of the obtained fluorescence intensity histogram corresponds, SNP in the region of interest in the gene of the subject can be detected.

Next, a measuring method for the case where there are two kinds of bases that could be substituted by SNPs. In this case, with regard to the gene of interest, beads A, beads B and beads C are prepared. Beads A have a probe covalently bonded to the surface thereof to which the nucleotide sequence without SNP will hybridize. Beads B has a probe covalently bonded to the surface thereof to which the nucleotide sequence of SNP in which a base at the site of polymorphism is substituted by a first base will hybridize. Beads C has a probe covalently bonded to the surface thereof to which the nucleotide sequence of SNP will hybridize in which the base at the site of polymorphism is substituted by a second base which is different from the first base. These three kinds of beads are mixed at different proportions, such as A:B:C=1:3:5, for example. Then, a target obtained by PCR-amplifying a fluorescence-labeled region of interest in the gene collected from the subject is added to the mixture of the three kinds of beads A, B and C and hybridized. Thereafter, the beads with the probe on the surface thereof to which the target has hybridized are measured by the flow cytometer.

Figs. 7A to 7F show possible patterns of the resultant fluorescence intensity histograms. Fig. 7A shows the case where a fluorescence marker was introduced into beads A that are fewest in number, namely when there is no SNPs in the gene of the subject. As mentioned above, when A:B:C=1:3:5, ideally the ratio of the count for peak 71a and that for peak 71b would be (3+5):1=8:1. Fig. 7B shows the case where the fluorescence marker was introduced into beads B, namely when there is SNP in the subject's gene by substitution with the first base. In this case, ideally the ratio of the count for peak 72a and that for peak 72b would be 6:3=2:1. Fig. 7C shows the pattern in the case where the fluorescence marker was introduced into beads C, namely when there is SNP in the subject's gene by substitution with the second base. In this case, ideally the ratio of the count for peak 73a and that for peak 73b would be 4:5.

Fig. 7D shows the pattern in the case where the fluorescence marker was introduced into beads A and beads B, namely when the gene of the subject contains a nucleotide sequence having no SNP and a nucleotide sequence having SNP due to substitution with the first base simultaneously. In this case, ideally the ratio of the count for peak 74a and that for peak 74b would be 5:4. Fig. 7E shows the pattern in the case where the fluorescence marker was introduced into beads A and beads C, namely when the gene of the subject contains a nucleotide sequence without SNP and a nucleotide sequence having SNP due to substitution with the second base simultaneously. In this case, ideally the ratio of the count for peak 75a and that for peak 75b would be 3:6=1:2. Fig. 7F shows the pattern in the case where the fluorescence marker was introduced into beads B and beads C, namely when the subject's gene contains a nucleotide sequence having SNP due to the first base and a nucleotide sequences having SNP due to substitution with the second base simultaneously. In this case, ideally the ratio of the count for peak 76a and that for peak 76b would be 1:8.

Thus, even when there are two kinds of bases that could possibly be substituted with SNP, the SNP in the region of interest in the subject's gene can be detected by determining to which of Figs. 7A to 7F the fluorescence intensity histogram pattern belongs. Even when there are three or more bases that could be substituted with SNP, it can be determined whether the subject's gene has SNP or, if it does, what kind of polymorphism that is, by the same method using the fluorescence intensity histograms. Specifically, beads having multiple targets bonded to the surface thereof are mixed at different proportions. The mixture is then mixed with a target obtained by PCR-amplifying a fluorescence-labeled nucleotide sequence in a region of the gene where SNP might be present and hybridized. Then, the hybridized beads are measured by the flow cytometer.

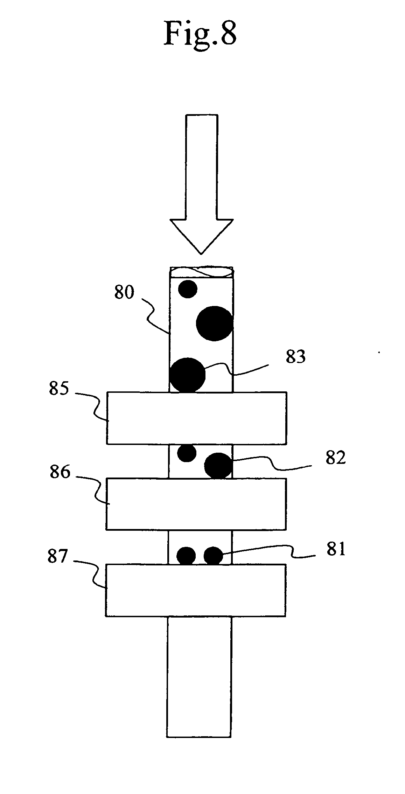

Fig. 8 schematically shows a system for recovering beads and biopolymers that are interacting on the surface of the beads after measurement in the flow cytometer. This system comprises filters 85, 86 and 87 having different meshes arranged in multiple stages in a flow path 80 of the flow cytometer. For example, when beads of three grain sizes of 4.5µm, 6.0µm and 10.0µm are used, filter 85 with a mesh size of 8µm, filter 86 with a mesh size of 5µm, and filter 87 with a mesh size of 3µm are arranged in this order from the upstream side. This arrangement of the filters 85 to 87 at the discharge outlet that are appropriately chosen for the grain sizes of the beads makes it possible to recover the beads, which, together with the solution, are normally discharged as waste fluid after completion of measurement in the flow cytometer. As shown, the beads of 4.5µm grain size pass through the filters 85 and 86 but captured by the filter 87 of 3µm mesh disposed most downstream. The beads of 6.0µm grain size pass through the filter 85 of 8µm mesh but captured by the filter 86 of 5µm mesh. The beads of 10.0µm grain size are captured by the filter 85 of 8µm mesh disposed most upstream.

Thus, in accordance with the invention, beads can be identified by a simple method and multiple kinds of specimens can be simultaneously measured. The invention also makes it possible to determine SNPs of DNA nucleotide sequences by a simple method.

## Claims

1. A target detection method for detecting an SNP in a DNA nucleotide sequence, said method comprising the steps of:
effecting a hybridization reaction between a DNA nucleotide sequence and a probe by mixing, in a sample solution containing a fluorescence-labeled DNA nucleotide sequence, beads of a first grain size having a first probe immobilized on the surface thereof which first probe hybridizes with a DNA nucleotide sequence having a site of single nucleotide polymorphism (SNP), and beads of a second grain size having a second probe which is different from the first probe immobilized on the surface thereof, and which second probe hybridizes with said DNA having a site of single nucleotide polymorphism (SNP), wherein, however, one base is substituted by a different base at the site of single nucleotide polymorphism (SNP),
detecting, after the hybridization reaction between said probes and said DNA nucleotide sequences, forward scatter and fluorescence simultaneously by flowing the beads in a flow cytometer, and
identifying the grain size of the beads based on the intensity of the forward scatter.

## Patentansprüche

1. Ziel-Nachweisverfahren zum Nachweisen eines SNP in einer DNA-Nukleotidsequenz, wobei das Verfahren die Schritte umfaßt:
Durchführen einer Hybridisierungsreaktion zwischen einer DNA-Nukleotidsequenz und einer Sonde durch Mischen, in einer Probenlösung, enthaltend eine Fluoreszenz-markierte DNA-Nukleotidsequenz, von Kügelchen einer ersten Korngröße, die eine erste Sonde auf ihrer Oberfläche immobilisiert haben, wobei die erste Sonde mit einer DNA-Nukleotidsequenz hybridisiert, die eine Stelle eines Einzel-Nukleotid-Polymorphismus (SNP) hat, und von Kügelchen einer zweiten Korngröße, die eine zweite Sonde auf ihrer Oberfläche immobilisiert haben, wobei die zweite Sonde von der ersten Sonde unterschiedlich ist, und wobei die zweite Sonde mit besagter DNA hybridisiert, die eine Stelle eines Einzel-Nukleotid-Polymorphismus (SNP) hat, wobei jedoch eine Base durch eine unterschiedliche Base an der Stelle des Einzel-Nukleotid-Polymorphismus (SNP) substituiert ist,
zeitgleiches Nachweisen, nach der Hybridisierungsreaktion zwischen den Sonden und den DNA-Nukleotidsequenzen, von einer Vorwärtsstreuung und Fluoreszenz, indem man die Kügelchen in einem Fluß-Cytometer fließen läßt, und
Identifizieren der Korngröße der Kügelchen, basierend auf der Intensität der Vorwärtsstreuung.

## Revendications

1. Procédé de détection de cible pour détecter un SNP dans une séquence de nucléotides d'ADN, ce procédé comprenant les étapes suivantes :
effectuer une réaction d'hybridation entre une séquence de nucléotides d'ADN et une sonde en mélangeant, dans une solution échantillon contenant une séquence de nucléotides d'ADN marquée par fluorescence, des billes d'une première dimension de grain ayant une première sonde immobilisée sur leur surface, cette première sonde s'hybridant avec une séquence de nucléotides d'ADN ayant un site de polymorphisme unique de nucléotides (SNP), et des billes d'une seconde dimension de grain ayant une seconde sonde différente de la première sonde immobilisée sur leur surface, la seconde sonde s'hybridant avec l'ADN ayant un site de polymorphisme unique de nucléotides (SNP), dans lequel toutefois une base est remplacée par une base différente au niveau du site de polymorphisme unique de nucléotides (SNP) ;
détecter, après la réaction d'hybridation entre les sondes et les séquences de nucléotides d'ADN, une dispersion et une fluorescence vers l'avant simultanément en faisant s'écouler les billes dans un cytomètre ; et
identifier une dimension de grain des billes sur la base de l'intensité de la dispersion vers l'avant.
